# EUROPEAN PATENT APPLICATION

(11) **EP 4 663 176 A2**
(43) Date of publication of application: **17.12.2025**
(21) Application number: 25213407.7
(22) Date of filing: 29.12.2021
(51) Int. Cl.: A61F 13/56

(54) **ABSORBENT ARTICLE WITH ADHESIVE PATTERN**

(62) Divisional of application: 21845075.7
(71) Applicant: Essity Hygiene and Health Aktiebolag, 405 03 Göteborg (SE)
(72) Inventor: Dahl, Annika, 40503 Göteborg (SE); Nyström, Anna-Karin, 405 03 Göteborg (SE); Ekstedt, Sofia, 405 03 Göteborg (SE); Blomström, Philip, 405 03 Göteborg (SE)
(74) Representative: Nederlandsch Octrooibureau

(57) **Abstract**

An absorbent article having a body-facing surface and a garment-facing surface and comprising a top sheet, a back sheet, and an absorbent core and an adhesive applied to the garment facing side of back sheet as adhesive stripes oriented along the transversal axis, wherein the adhesive stripes extend over transversal width of the absorbent core. The absorbent article is intended to be folded along transversal imaginary fold lines including a first imaginary fold line arranged in a first fold region positioned between two adhesive stripes and a second imaginary fold line arranged in a second fold region positioned between two adhesive stripes, and no adhesive is present in the first and second fold regions. Further, respective longitudinal widths of the first and second fold regions are larger than the average longitudinal distance between the adhesive stripes and the width of the first fold region differs from the width of the second fold region.

## Description

### TECHNICAL FIELD OF THE INVENTION

The disclosure relates to an absorbent article such as a sanitary napkin or an incontinence protector, having a length extending in a longitudinal direction along a longitudinal axis and a width extending in transversal direction along a transversal axis and having a body-facing surface and a garment-facing surface, the absorbent article comprising a top sheet, a back sheet, and an absorbent core arranged between the top sheet and the back sheet, and an adhesive applied to the garment facing side of back sheet as adhesive stripes oriented along the transversal axis.

### BACKGROUND OF THE INVENTION

Absorbent articles, such as a sanitary napkin or an incontinence protector, are commonly folded twice and wrapped before use, and unwrapped, unfolded and placed in the user's undergarment during use. The back sheet of such absorbent articles are therefore provided with attachment adhesive which will facilitate attachment of the absorbent article to the wearers undergarment, and hold it in place during use.

The attachment adhesive is protected before use by either a release paper which covers the adhesive pattern or a wrapper which covers the adhesive pattern and also encloses the entire absorbent article.

The adhesive which is used for adhering an absorbent article to an undergarment may for example be a hot melt type adhesive. Hot melt adhesives are solid at room temperature, but transform into a fluid or melt form upon heating to a certain temperature. Furthermore, hot melt adhesives are thermoplastic compositions, so that the transformation from solid form to fluid form is reversible. When a hot melt adhesive is to be applied to an absorbent article, the hot melt adhesive is first heated to a temperature at which it takes a fluid form, after which it can be applied to the back sheet of the absorbent article. After application to the absorbent article, the hot melt adhesive transforms into solid form as it cools. During cooling, bonding takes place to the absorbent article.

Furthermore, the adhesive which is used for adhesion of an absorbent article to an undergarment may also be a pressure-sensitive type adhesive. A pressure-sensitive adhesive applied to an absorbent article may be made to attach and thereby bond to an undergarment via application of pressure, i.e. activation with heat or other means is not required to achieve bonding to an undergarment.

Today, it is common to utilize an adhesive which is both pressure-sensitive and a hot melt type adhesive, i.e. an adhesive which is a pressure-sensitive hot melt adhesive, for attachment to an undergarment. The pressure-sensitive hot melt adhesive is thereby bonded to the back sheet of an absorbent article upon application, as described above for hot melt adhesives, and the pressure-sensitive hot melt adhesive can thereafter in use be fastened to an undergarment via application of pressure, as described above for pressure-sensitive adhesives. Alternatively, the pressure-sensitive hot melt adhesive can be applied to the wrapper and transferred to the back sheet of the absorbent article as the wrapper is provided to the absorbent article. When the wrapper is removed before use of the article, the pressure-sensitive hot melt adhesive, which has bonded to the back sheet, adheres to the back sheet and can thereafter in use be fastened to an undergarment via application of pressure.

Reliable anchorage of the adhesive to the back sheet of the absorbent article is key so that a reliable attachment to the undergarment can be obtained during use. If not, there is a risk that the adhesive unsticks from the back sheet layer and fastens to the undergarment.

Manufacturers of absorbent articles are always looking for ways to make production more reliable, simpler and to reduce cost. One aspect of this is to use thinner materials, for cost and environmental reasons. However, thinner wrappers are more fragile during manufacturing. Reducing the amount of adhesive needed while still achieve acceptable adherence of the product is also desirable.

### SUMMARY OF THE INVENTION

It is an object of the invention to overcome the above mentioned issues with using just the right amount of adhesive in order for the back sheet and release paper or wrapper to be unaffected but at the same time allow for good adherence to the undergarment during use. It has been seen for example that thinner materials will easily wrinkle or rupture during manufacturing in the area around the adhesive pattern, and that the article is particularly vulnerable to this in the areas where the article is folded before use.

The objects above are achieved by the features of the independent claim.

Further advantages are achieved by implementing one or several of the features described herein.

The disclosure relates to an absorbent article, such as a sanitary napkin or an incontinence protector, having a length extending in a longitudinal direction along a longitudinal axis and a width extending in transversal direction along a transversal axis and having a body-facing surface and a garment-facing surface. The absorbent article comprises a top sheet, a back sheet, and an absorbent core arranged between the top sheet and the back sheet, and an adhesive applied to the garment facing side of the back sheet as adhesive stripes oriented along the transversal axis. The adhesive stripes extend over transversal width of the absorbent core. The adhesive stripes form at least three adhesive pattern regions; a first adhesive pattern region is formed by adjacent adhesive stripes arranged at a front portion of the absorbent core, a second adhesive pattern region is formed by adjacent adhesive stripes arranged at a rear portion of the absorbent core and a third adhesive pattern region is formed by adjacent adhesive stripes arranged at a central portion of the absorbent core.

The average transversal length of the adhesive stripes and/or the average longitudinal width of the adhesive stripes and/or the number of adjacent adhesive stripes differ between at least two of the adhesive pattern regions.

The adhesive stripes forming an adhesive pattern region are adjacent one another, or with other words neighboring each other, i.e. are arranged next to each other with adhesive free space in between.

Each adhesive stripe has an adhesive stripe area, and the sum of the adhesive stripe areas within one adhesive pattern region differs by no more than 19% from the sum of the adhesive stripe areas within another adhesive pattern region.

In one example the sum of the adhesive stripe areas within one adhesive pattern region differs by no more than 10% from the sum of the adhesive stripe areas within another adhesive pattern region.

The sum of the adhesive stripe areas may be substantially the same in all adhesive pattern areas, i.e. the sum of adhesive stripe areas in one pattern region corresponds to the sum of adhesive stripe areas in any other pattern region.

Having a total adhesive stripe area correspond between each pattern and differ by no more than 19%, preferably no more than 10%, and most preferably by no more than 5% is achieved by adjusting the length and width of the adjacent adhesive stripes, and the number of adhesive stripes in each pattern.

By having the same adhesive stripe area in each adhesive pattern region the same amount of adhesive can be applied in each pattern region which simplifies the application during manufacturing. The adhesive is preferably applied by slot technique and with the adhesive pattern as disclosed herein consistent amount of adhesive can be applied throughout the application step.

According to one aspect the adjacent, or neighboring, adhesive stripes arranged within the same adhesive pattern region are uniform, i.e. are duplicates of each other which facilitates the application of the adhesive during manufacturing. Each adhesive stripe has a length extending along the transversal axis, a width extending along the longitudinal axis, and the lengths and the widths of the adhesive stripes arranged within the same adhesive pattern region are essentially the same, and differs by no more than 10%.

As mentioned, if the sum of all adhesive stripe areas present within each respective adhesive pattern region is the same then the total surface weight of adhesive applied in each respective adhesive pattern region can be the same, or at least differ by no more than 10% between the adhesive pattern regions.

As an example, the surface weight of the adhesive applied in each adhesive pattern region may be 10-25 gsm.

The adhesive is preferably a PSHM.

According to one aspect the adhesive stripes are arranged to overlap the absorbent core such that at least 90% of each adhesive stripe area is overlapping the absorbent core.

The adhesive stripes in each adhesive pattern region are chosen to extend substantially over the whole width of the absorbent core and extend beyond the transversal width of the absorbent core by no more than 10% of the transversal length of the adhesive stripe. Thereby, the adhesive stripes follow the contours of the core even if the core is non-rectangular. Put in other words, the length of the adhesive stripes is preferably 70-120% of the width of the absorbent core in transversal direction, more preferably 80 - 110 % of the width of the absorbent core in transversal direction, and most preferably 90 - 105 % of the width of the absorbent core in transversal direction. By allowing the width and length of the adhesive stripes to differ between the different adhesive pattern regions the contours of the adhesive pattern regions can follow the contours of the core, and while still having the same total adhesive stripe area within each adhesive pattern region the same amount of adhesive can be applied in the regions which facilitates manufacturing.

By arranging the adhesive stripes to overlap the core, and to extend laterally outside of the core contour by no more than 10% of the transversal length of the adhesive stripe, an even application pressure of the adhesive during the application to the absorbent article can be achieved. This is especially important for absorbent articles comprising an absorbent core since the thickness along the transversal width of such absorbent articles differs.

The top sheet and the back sheet forms protruding portions extending out from the edges of the absorbent core. The thickness, in z-direction, of the protruding portions is less than the thickness of absorbent product where the absorbent core is present. Hence, a more consistent and reliable adherence of the adhesive stripes to the back sheet is achieved if the adhesive stripes is arranged to extend across the width of the absorbent core but does substantially not extend out onto the protruding portions.

The above mentioned arrangement of the adhesive stripes in relation to the absorbent core also helps avoid wrinkles, ruptures and other problems which can occur at the transition area between thick and thin when the cover material, such as a release paper or wrapper, is applied to the absorbent product, especially when using a thin material. Also, a thin wrapper material is for example prone to wrinkle or rupture as the adhesive is applied to it by slot technique. The cover material may have thickness of no more than 20µm, preferably no more than 17 µm in Z-direction.

In addition, the adhesive pattern will allow for a more consistent and reliable attachment of the absorbent article to the undergarment when in use.

According to one aspect, 30-70%, preferably 45-65%, of the absorbent core area is covered by the adhesive stripes, which facilitates good adherence of the absorbent article to the user's undergarment during use.

According to one example, a fourth adhesive pattern region is arranged in a central portion of the absorbent core, and the fourth adhesive pattern region is arranged closer to the rear portion than the third adhesive pattern region.

The adhesive stripes of the first adhesive pattern region may have the same length and the same width as the adhesive stripes in the third adhesive pattern region, and the adhesive stripes of the second adhesive pattern region may have the same length and the same width as the adhesive stripes in the fourth adhesive pattern region. The number of adhesive stripes in the first adhesive pattern region may be the same as the number of adhesive stripes in the third adhesive pattern region, and the number of adhesive stripes in the second adhesive pattern region may be the same as the number of adhesive stripes in the fourth adhesive pattern region.

The number of uniform adjacent adhesive stripes forming an adhesive pattern region may comprise at least two adhesive stripes.

The number of uniform adjacent adhesive stripes forming an adhesive pattern region may comprise no more than three adhesive stripes.

The longitudinal distance between the adhesive stripes forming an adhesive pattern region is the same, whereas they may differ between the adhesive pattern zones, and the distance between two adhesive pattern zones may also differ, and be longer or shorter than the distance between the adhesive stripes within an adhesive pattern zone. The longitudinal distance between the adhesive stripes may be 20-60% of the longitudinal width of the adhesive stripes within an adhesive pattern zone.

The adhesive pattern regions may be evenly distributed along the length of the absorbent core, such that each adhesive pattern region is equal in length in longitudinal direction. The adhesive stripes are thereby evenly distributed along the length of the absorbent core, and facilitates an even adherence to the garment during use.

The rear of the absorbent core may be wider than the other parts of the core. The adhesive stripes may therefore be wider and longer but fewer in the second adhesive pattern region, i.e. at the rear portion of the absorbent core, to cover the width of the absorbent core well. The longitudinal distance between the adhesive stripes may be longer in the second adhesive pattern region than in the first adhesive pattern region.

The absorbent core may also be non-continuous in transversal direction in the rear portion of the absorbent article such as to form an intermittent core-free part, and in such case the adhesive stripes of the second adhesive pattern region may covers both the absorbent core and the intermittent core-free part of the rear portion in transversal direction.

The absorbent article is intended to be folded along transversal imaginary fold lines. A first imaginary fold line is arranged in a first fold region positioned between two adhesive stripes, and a second imaginary fold line is arranged in a second fold region positioned between two adhesive stripes, and no adhesive is present at the first and second fold region. The first fold region and/or the second fold region may coincide with the adhesive free region at the transition between two adhesive pattern regions, i.e. between an adhesive stripe of one adhesive pattern region and an adhesive stripe of another adjacent adhesive pattern region.

The respective longitudinal widths of the first and second fold regions are larger than the average longitudinal distance between the adhesive stripes. The larger width allows for folding of the product at the imaginary fold lines without interfering with the adhesive stripes. Folding the product at an adhesive free region reduces the risk of affecting the material at which the adhesive is present.

As an example the longitudinal width of the first fold region is at least 4 mm, preferably 5-15 mm, and the longitudinal width of the second fold region is at least 4 mm, preferably 5-15 mm in longitudinal direction. This allows for an absorbent article of average thickness to fold without the presence of adhesive at the fold.

The disclosure also relates to an absorbent article in which the absorbent article is intended to be folded along transversal imaginary fold lines and wherein a first imaginary fold line is arranged in a first fold region positioned between two adhesive stripes, and a second imaginary fold line is arranged in a second fold region positioned between two adhesive stripes, and wherein no adhesive is present at the first and second fold region. This is an alternative and/or complementary way of solving the technical problem set out in this disclosure, i.e. to reduce the amount of adhesive used and to use thinner materials while still maintaining good performance of the absorbent article.

The absorbent article is, as also disclosed earlier, a sanitary napkin or an incontinence protector, having a length extending in a longitudinal direction along a longitudinal axis and a width extending in transversal direction along a transversal axis and having a body-facing surface and a garment-facing surface, the absorbent article comprising a top sheet, a back sheet, and an absorbent core arranged between the top sheet and the back sheet, and having an adhesive applied to the garment facing side of the back sheet as adhesive stripes oriented along the transversal axis, wherein the adhesive stripes extend over transversal width of the absorbent core.

An absorbent article comprising the fold regions as disclosed above may, in combination, also comprise the adhesive pattern regions disclosed above and the specified features disclosed in combination with the adhesive pattern regions and the adhesive stripes. In particular adhesive pattern regions in which the adhesive stripe has an adhesive stripe area, and the sum of the adhesive stripe areas within one adhesive pattern region differ by no more than 19% from the sum of the adhesive stripe areas within another adhesive pattern region.

The respective longitudinal widths of the first and second fold region are larger than the average longitudinal distance between the adhesive stripes, wherein the width of the first fold region differs from the second fold region, and wherein at least one of the first and the second fold region has a width of 5-15mm. This will allow the absorbent article to be folded twice without interference of adhesives at the fold regions.

The adhesive stripes of the absorbent articles disclosed herein extend across the width of the absorbent core and their length substantially corresponds to the width of the absorbent core. That is, each adhesive stripe extends beyond the transversal width of the absorbent core by no more than 10% of the transversal length of the adhesive stripe. The adhesive stripes are arranged such that 30-70%, preferably 45-65%, of the absorbent core area is covered by the adhesive stripes.

The shape of the absorbent core may be non-rectangular, i.e. the transversal width of the core varies along its longitudinal length. The length of the stripes is preferably 70-120% of the width of the absorbent core in transversal direction, more preferably 80 - 110 % of the width of the absorbent core in transversal direction, and most preferably 90 - 105 % of the width of the absorbent core in transversal direction such that the width of the adhesive pattern regions corresponds to side contour of absorbent core.

The average width of the absorbent core may also differ between at least between two of the front portion, rear portion, and central portion. The average width of the adhesive stripes of each respective adhesive pattern region therefore differs in order to allow the side contours of the adhesive pattern regions, set by the width of the adhesive stripes, to follow the side contour for the absorbent core. Thereby the width of the adhesive pattern regions varies.

The longitudinal length of the front portion may be at least 10%, 15%, 20% or 25% of the total longitudinal length of the absorbent article. The longitudinal length of the rear portion may be at least 10%, 15%, 20% or 25% of the total longitudinal length of the absorbent article.

The absorbent article may be provided with a cover material covering at least the adhesive pattern regions, the cover material comprising a material having a basis weight of 10-25 gsm. The cover material is a release paper or a single wrap. The cover material may be biobased.

The adhesive pattern as disclosed herein allows for less adhesive to be used while still maintaining good performance during use, which is particularly beneficial when using thinner cover materials.

Moreover, biobased materials are in general more fragile, and also often chosen to be thinner due to cost, hence the possibility to use reduce the amount of adhesive is beneficial when using a biobased wrapper or release paper.

The absorbent article may have a variety of different outer contours, i.e. it may be provided with symmetrical or non-symmetrical wings, it may comprise a neck or its width may be uniform along the longitudinal axis.

Another object of the present disclosure is to provide a method for production of an absorbent article such as a sanitary napkin or an incontinence protector which overcomes the issues set out above. Therefore, further disclosed herein is a method of manufacturing an absorbent article as set out above, the absorbent article being a sanitary napkin or an incontinence protector having a length extending in a longitudinal direction along a longitudinal axis and a width extending in transversal direction along a transversal axis and having a body-facing surface and a garment-facing surface, the absorbent article comprising a top sheet, a back sheet, and an absorbent core arranged between the top sheet and the back sheet.

The method comprises applying an adhesive to the garment facing side of back sheet as adhesive stripes oriented along the transversal axis such the adhesive stripes extend over transversal width of the absorbent core, arranging adhesive stripes to form at least three adhesive pattern regions and forming a first adhesive pattern region by applying adhesive stripes at a front portion of the absorbent core, forming a second adhesive pattern region by applying adhesive stripes at a rear portion of the absorbent core and forming a third adhesive pattern region by applying adhesive stripes at a central portion of the absorbent core. The adhesive is applied such that the average transversal length of the adhesive stripes and/or the average longitudinal width of the adhesive stripes and/or the number of adhesive stripes differ between at least two of the adhesive pattern regions. The method further comprises applying adhesive surface weight to each adhesive stripe such that the sum of adhesive surface weight applied within one adhesive pattern region, differ by no more than 19% from the sum of adhesive surface weight applied within another adhesive pattern region.

### BRIEF DESCRIPTION OF THE DRAWINGS

The present invention is illustrated by way of examples and not limited to the accompanying drawings, in which like references indicate similar elements:
FIG. 1 schematically illustrates an absorbent article in accordance with the present disclosure;
FIGS. 2a-b schematically illustrates absorbent articles with alternative outer contours as compared with FIG 1;
FIG 3. schematically illustrates an absorbent article according to an alternative aspect in accordance with the present disclosure;
FIG 4. schematically illustrates an absorbent article according to an alternative aspect in accordance with the present disclosure;
FIG 5a. schematically illustrates an absorbent article according to an alternative aspect in accordance with the present disclosure;
FIG 5b. schematically illustrates an absorbent article according to an alternative aspect in accordance with the present disclosure; and
FIG 6. schematically illustrates absorbent articles with alternative absorbent core contour as compared with FIG 1;

### DETAILED DESCRIPTION OF THE DRAWINGS

It is to be understood by one of ordinary skill in the art that the following is a description of exemplary embodiments only, and is not intended as limiting the broader aspects of the present disclosure.

Different aspects of the present disclosure will be described more fully hereinafter with reference to the enclosed drawings. The embodiments disclosed herein can, however, be realized in many different forms and should not be construed as being limited to the aspects set forth herein.

It is to be understood that the drawings are schematic and that individual components, such as layers of material are not necessarily drawn to scale.

All Figures show an absorbent article 11 with the garment facing side of the back sheet up. The back sheet is provided with a striped adhesive pattern.

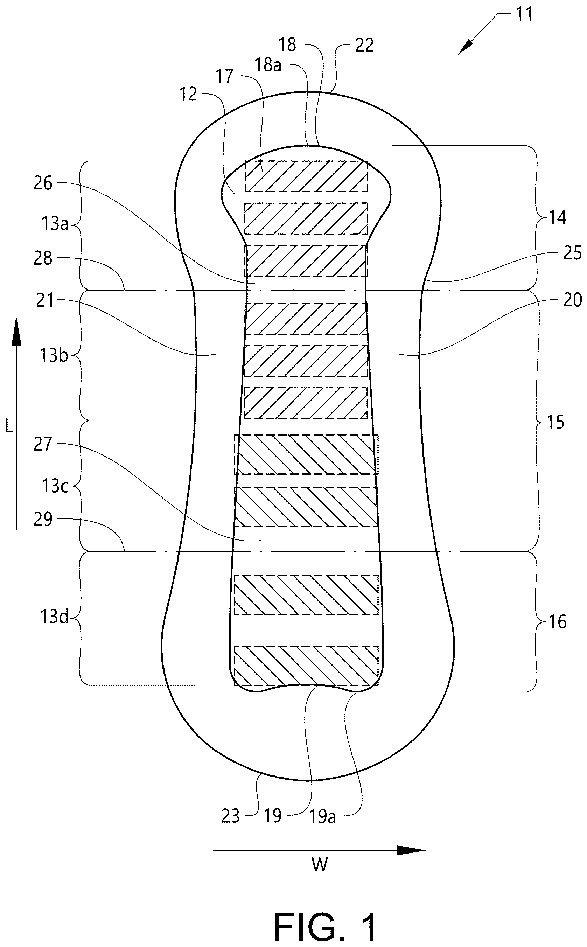

Figure 1 show a plan view of a first embodiment of an absorbent article 11, such as a sanitary napkin, or an incontinence protector, according to the present invention. The absorbent article 11 has a conventional construction and comprises a liquid-permeable top sheet, a liquid-impermeable back sheet, and an absorbent structure arranged between the top sheet and the back sheet. In use, the liquid-permeable top sheet is intended to face a user, and the liquid-impermeable back sheet is intended to face a user's undergarment.

Usually, the liquid-permeable top sheet and the liquid-impermeable back sheet extend out from the edges of the absorbent core 12 and protruding portions 20, 21 of the top sheet and the back sheet are thereby joined to one another around the periphery of the absorbent core, e.g. by gluing, sewing or by welding with heat or ultrasound. Hence, the thickness of the absorbent article is less in these protruding portions 20, 21 than in the portion where the absorbent core 12 is present.

The absorbent article 11 has a conventional, essentially elongated shape, and has a longitudinal direction L and a transverse direction W. The absorbent article 11 has a front 22 and a rear 23 and the absorbent core 12 is usually arranged in the central part of the absorbent article 11, i.e. what is also called the "crotch area".

The material in the liquid-permeable top sheet may be of any suitable type, and can for instance comprise a non-woven material, e.g. spunbond, meltblown, carded material, hydroentangled material, wet-laid material etc. Suitable non-woven materials can be composites of natural fibres, cotton fibres, artificial fibres such as polyester, polyethylene, polypropylene, viscose etc., or a mixture of natural and artificial fibres. Furthermore, the material in the liquid-permeable top sheet may comprise a material of tow fibres, which can be bound to each other in a bonding pattern. Further examples of materials which are suitable for the liquid-permeable top sheet are perforated plastic films, nets and open-cell or perforated foam materials. Different types of laminates, e.g. laminates of non-woven material and plastic film may also be used. Materials which are suitable to use for the liquid-permeable top sheet should be soft and nonirritating to the skin, and should be readily penetrated by bodily fluids, e.g. urine and menstrual fluid. However, it is not necessary that the liquid- permeable top sheet is a separate material layer, but rather the top sheet can instead be an integrated portion of the absorbent structure core, a material especially intended for admission of fluid is arranged centrally, and a specially soft and comfortable material is arranged along the longitudinal side edges of the article 11.

The liquid-impermeable back sheet of the absorbent article 11 according to the invention comprises an outer fibrous material layer having an outer surface which is intended to lie against a user's undergarment. Examples of different types of materials which may be used for the liquid impermeable back sheet are a non-woven material which has been made liquid-impermeable through coating or treatment with a liquid-impermeable material, a hydrophobic non-woven material which resists liquid penetration or a laminate of a plastic film and a non-woven material. In order that the absorbent article 11 should feel airy and comfortable to wear, the liquid-impermeable back sheet suitably allows air and water vapour to be transported out from the absorbent core 12. Examples of materials which are breathable and which are suitable for use as the liquid-impermeable back sheet 3 are laminates of non-woven materials, laminates of porous polymer films and non-woven materials. The liquid-impermeable back sheet 3 can be elastic or inelastic. The non-woven material can be spunbond, meltblown, carded, hydroentangled materials or the like. Preferred materials for the non-woven material are polyethylene, polypropylene and the like, although other compositions of natural or synthetic material may also be used. If the liquid-impermeable back sheet is a laminate of nonwoven-materials, it can for example be an SM-laminate, i.e. a laminate comprising one component layer of spunbond and one component layer of meltblown, an SMS-laminate, i.e. a laminate comprising two component layers of spunbond and one component layer of meltblown placed between the two component layers of spunbond, an SMMS-laminate, i.e. a laminate comprising two component layers of spunbond and two component layers of meltblown placed between the two component layers of spunbond, an SMM-laminate, i.e. a laminate with the spunbond-meltblown-meltblown configuration, or an SMSM-laminate, i.e. a laminate with the spunbond-meltblown- spunbond-meltblown configuration.

Furthermore, an adhesive is applied to the garment facing side of back sheet as adhesive stripes 17 oriented along the transversal axis (W). The adhesive stripes 17 extend over transversal width of the absorbent core, and does not substantially extend over the protruding portions of the absorbent article 11. The adhesive stripes 17 are intended to adhere to a user's undergarments in use, and are preferably PSHM.

The adhesive stripes 17 of the absorbent article 11 in Figure 1 are arranged in four adhesive pattern regions 13a-d. A first adhesive pattern region 13a, formed by three adjacent and uniform adhesive stripes 17, is arranged in a front portion 14 of the absorbent core 12. A second adhesive pattern region 13d, formed by two adjacent and uniform adhesive stripes 17, is arranged at a rear portion 16 of the absorbent core 12. A third adhesive pattern region 13b is formed by three adjacent and uniform adhesive stripes 17 arranged at the frontmost part of the central portion 15 of the absorbent core 12. The adhesive stripes 17 of the third adhesive pattern region 13b is in this example uniform to the adhesive stripes 17 arranged in the first adhesive pattern region 13a. The central portion 15 of the absorbent core 12 further comprises a fourth adhesive pattern region 13c, formed by two adjacent and uniform adhesive stripes 17. The adhesive stripes 17 of the fourth adhesive pattern region 13c is in this example uniform to the adhesive stripes 17 arranged in the second adhesive pattern region 13d.

The uniform adhesive stripes 17 of each respective adhesive pattern region 13a-d has the same length in transversal direction W and width in longitudinal direction L, and thereby also have the same adhesive stripe area covering the absorbent core 12. The total sum of the adhesive stripe areas of the adhesive stripes arranged within each one of the four adhesive pattern regions are substantially the same.

The absorbent core 12 in Figure 1 is non-rectangular. It has a narrow neck 25 in the area of transition between front portion 14 and central portion 15, which is the narrowest part of the absorbent core 12. The front portion 14 is in average wider than the central portion 15 and the rear portion 16. The rear portion 16 and the central portion 15 is narrowing towards the neck 25 of the absorbent core 12. The length of the adhesive stripes 17 is 70-120% of the width of the absorbent core in transversal direction W such as to substantially cover the width of the absorbent core 12 without overlapping the protruding portions 20, 21.

The absorbent core 12 has a front edge 18 arranged at the front portion 14 of the absorbent core, and the front edge 18 comprises a front point 18a being the frontmost point of the front edge in longitudinal direction. In Figure 1, the adhesive stripes of the first adhesive region 13a does not extend beyond the front point 18a. In general according to this disclosure the adhesive stripes of the first adhesive region 13a may extend no further in longitudinal direction than 5mm beyond the front point 18a.

The absorbent core has a rear edge 19 arranged in at rear portion 16 of the absorbent core, and the rear edge 19 comprises a rear point 19a being the rearmost point of the rear edge in longitudinal direction. In Figure 1 the adhesive stripes 17 of the second adhesive region 13d does not extend beyond the rear point 19a. In general according to this disclosure the adhesive stripes of the second adhesive region 13d may extend no further in longitudinal direction than 5mm beyond the rear point 19a.

In order to achieve adherence of the adhesive stripes 17 to the absorbent article 11, and then equally good adherence of the absorbent article 11 to the user's undergarment during use, the total adhesive coverage of absorbent core area should be 30-70%. The example shown in Figure 1 represents an adhesive coverage of the absorbent core area of about 60%.

The absorbent article comprises two fold regions arranged between the front portion and the central portion of the absorbent core and between the central portion and the rear portion of the absorbent core respectively. These fold regions are adhesive free. A first imaginary fold line 28 is positioned in the first fold region 26, and a second imaginary fold line 29 is arranged in a second fold region 27.

The first fold region 26 and the second fold region 27 coincide with the adhesive free region at the transition between two adhesive pattern regions. The first fold region 26, comprising the first imaginary fold line 28, is arranged between an adhesive stripe 17 of the first adhesive pattern region 13a and an adhesive stripe 17 of the third adjacent adhesive pattern region 13b. The second fold region 27, comprising the second imaginary fold line 29, is arranged between an adhesive stripe 17 of the second adhesive pattern region 13d and an adhesive stripe 17 of the fourth adjacent adhesive pattern region 13c.

The second fold region, arranged towards the rear 23 of the absorbent article, is wider in longitudinal direction L than the first fold region, arranged towards the front 22 of the absorbent article.

The absorbent article is intended to be folded and packaged in folded state, either in a separate package or by sealing the wrapper if it is provided with such. The absorbent article is intended to be folded at the first imaginary fold line 28 first such that the front region 14 is arranged on top of the central region 15, and then folded at the second imaginary fold line 29 such that the rear region 16 is arranged on top of the already folded front region 14, thereby stacked on top of both the central region 15 at the front region 14 in Z-direction. The area affected by the folding is thus larger at the second fold, which is at the second fold region in this example. The adhesive free second fold region 27, preferably 5-15mm in longitudinal width, allows absorbent article to be largely unaffected by the fold due to the lack of adhesive at the fold.

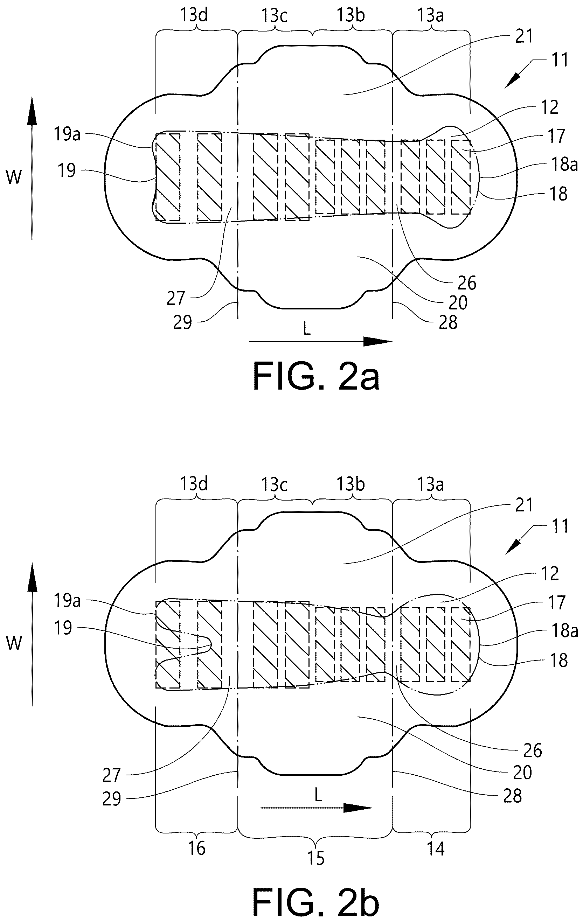

Figure 2a represents the same embodiment as shown in Figure 1, but with the addition of wings arranged along the sides of the absorbent article. The wings extend out from the protruding portions 20, 21.

Figure 2b shows an alternative embodiment of the absorbent core. The rear edge 19 of the rear portion 16 of the absorbent core in Figure 2b is indented such that the core is non-continuous in transversal direction W for most part of the rear portion 16 and thereby forms two core legs and an intermittent core-free part in between. The two adjacent adhesive stripes 17 span over the width of the core, thereby also spanning over the intermittent core-free part. The side contours of the adhesive stripes 17 thereby follow the contours of the absorbent core 12.

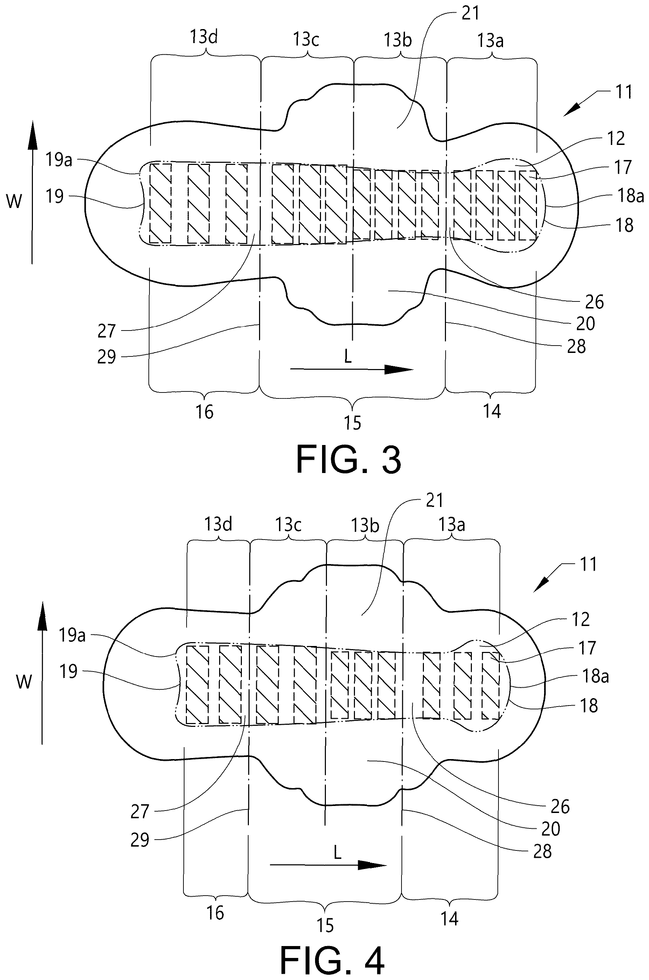

The adhesive stripes 17 of the absorbent article 11 in Figure 3 and Figure 4, likewise as in Figure 1, are arranged in four adhesive pattern regions 13a-d.

In Figure 3, a first adhesive pattern region 13a, formed by four adjacent and uniform adhesive stripes 17, is arranged in a front portion 14 of the absorbent core. A second adhesive pattern region 13d, formed by three adjacent and uniform adhesive stripes 17, is arranged at a rear portion 16 of the absorbent core 12. A third adhesive pattern region 13b is formed by four adjacent and uniform adhesive stripes 17 arranged at the frontmost part of the central portion 15 of the absorbent core 12. The adhesive stripes 17 of the third adhesive pattern region 13b is in this example uniform to the adhesive stripes 17 arranged in the first adhesive pattern region 13a. The central portion 15 of the absorbent core 12 further comprises a fourth adhesive pattern region 13c, formed by three adjacent and uniform adhesive stripes 17. The adhesive stripes 17 of the fourth adhesive pattern region 13c is in this example uniform to the adhesive stripes 17 arranged in the second adhesive pattern region 13d.

The absorbent core may have different shapes and lengths depending on the product type and the size of the absorbent article. In the example shown in Figure 3, the rear portion 16 of the absorbent core is elongated since the absorbent article is a longer type. The longitudinal length of the rear portion 16 is about 30% of the total length of the absorbent core.

The longitudinal distance between the adhesive stripes in the second pattern region 13d is longer than the longitudinal distance between the adhesive stripes of the fourth pattern region 13c.

As in Figure 1, the absorbent article of Figure 3 comprises two adhesive free fold regions 26, 27 comprising imaginary fold lines. The longitudinal width of the second fold region 27 is larger than the longitudinal width of the first fold region 26, such that the longitudinal distance between the adhesive stripes 17 neighboring the second fold region 27 is larger than the longitudinal distance between the adhesive stripes 17 neighboring the first fold region 26. Moreover, the longitudinal width of the first fold region 26 and second fold region 27 respectively is larger than the average longitudinal distance between the adhesive stripes 17 of the absorbent article.

In Figure 4, a first adhesive pattern region 13a, formed by three adjacent and uniform adhesive stripes 17, is arranged in a front portion 14 of the absorbent core. A second adhesive pattern region 13d, formed by two adjacent and uniform adhesive stripes 17, is arranged at a rear portion 16 of the absorbent core 12. A third adhesive pattern region 13b is formed by three adjacent and uniform adhesive stripes 17 arranged at the frontmost part of the central portion 15 of the absorbent core 12. The adhesive stripes 17 of the third adhesive pattern region 13b is in this example uniform to the adhesive stripes 17 arranged in the first adhesive pattern region 13a. The longitudinal distance between the adhesive stripes 17 of the first adhesive patters region 13a is longer than the longitudinal distance between the adhesive stripes 17 of the third adhesive patters region 13b. The central portion 15 of the absorbent core 12 further comprises a fourth adhesive pattern region 13c, formed by two adjacent and uniform adhesive stripes 17. The adhesive stripes 17 of the fourth adhesive pattern region 13c is in this example uniform to the adhesive stripes 17 arranged in the second adhesive pattern region 13d.

The uniform adhesive stripes 17 of each respective adhesive pattern region 13a-d in Figure 3 and Figure 4 has the same length in transversal direction W and width in longitudinal direction L, and thereby also have the same adhesive stripe area covering the absorbent core 12. The total sum of the adhesive stripe areas of the adhesive stripes 17 arranged within each one of the four adhesive pattern regions 13a-d are substantially the same. The length of the adhesive stripes 17, the width of the adhesive stripes 17 and the number of adhesive stripes 17 are set to follow the contour of the absorbent core 12, to give an even distribution of adhesive across the absorbent core 12 while still maintaining the same sum of adhesive stripe areas between the adhesive pattern regions 13a-d.

In Figure 4, the longitudinal width of the first fold region 26 is larger than the longitudinal width of the second fold region 27. The longitudinal width of the first and second fold regions differ, but both are larger than the average longitudinal distance between the adhesive stripes 17 of the absorbent article.

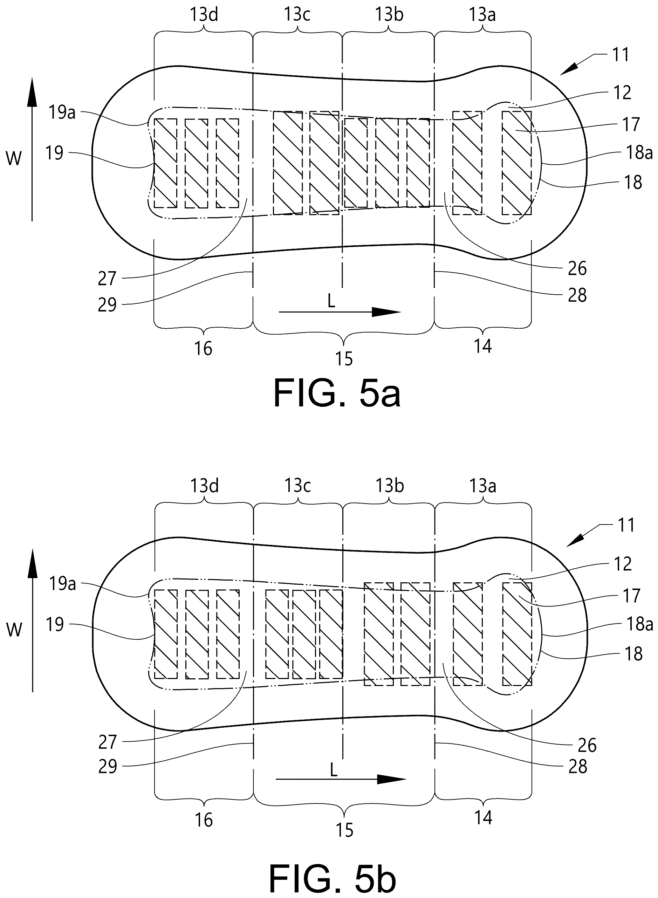

Figure 5a and 5b show other examples of how adhesive stripes 17 may be arranged to form four adhesive pattern regions 13a-d according to aspects as disclosed herein.

In Figure 5a the first adhesive pattern region 13a and fourth adhesive pattern region 13c comprises two uniform adhesive stripes 17 of equal length and width respectively, and the second adhesive pattern region 13d and third adhesive pattern region 13b comprises three uniform adhesive stripes 17 of equal length and width respectively. In Figure 5b the first adhesive pattern region 13a and third adhesive pattern region 13b comprises two uniform adhesive stripes 17 of equal length and width respectively, and the second adhesive pattern region 13d and fourth adhesive pattern region 13c comprises three uniform adhesive stripes 17 of equal length and width respectively.

The total sum of the adhesive stripe areas of the adhesive stripes arranged within each one of the four adhesive pattern regions are substantially the same in Figures 5a-b.

The adhesive stripes 17 extend over the transversal width of the absorbent core 12, and as illustrated in Figure 5a the adhesive stripes 17 may have a slightly shorter transversal length than the width of the absorbent core 12, or extend slightly beyond the absorbent core in transversal direction. The adhesive stripes 17 all have a length which is 70-120% of the of the width of the absorbent core 12 in transversal direction.

Both Figure 5a and Figure 5b comprises two fold regions 26, 27 which differ in longitudinal width from each other but have a longitudinal width which is larger than the average distance between the adhesive stripes 17 of the absorbent product.

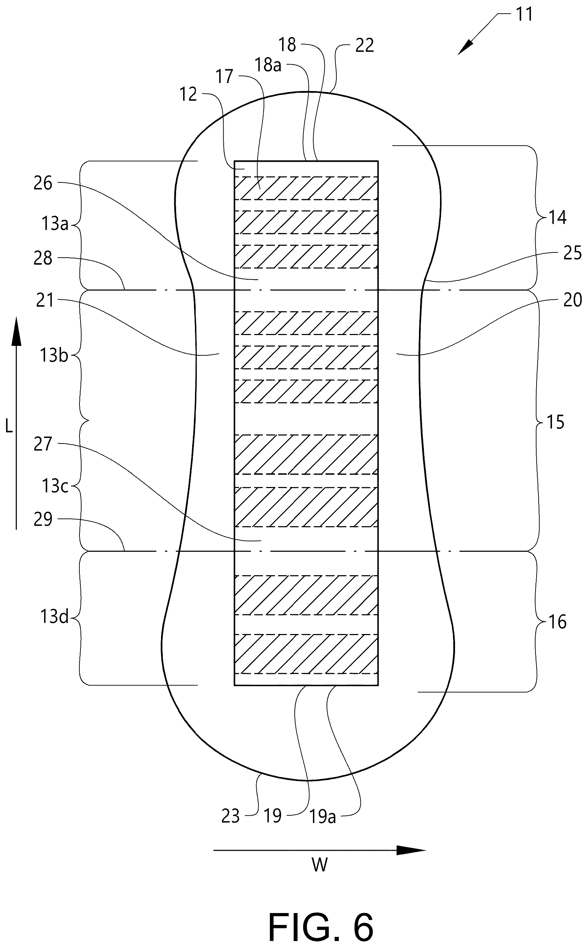

Figure 6 shows an absorbent article with an alternative absorbent core 12 as compared to Figure 1. The absorbent core in Figure 6 is uniform, rectangular in shape.

The adhesive stripes 17 in Figure 6 are arranged in four adhesive pattern regions 13a-d. A first adhesive pattern region 13a, formed by three adjacent and uniform adhesive stripes 17, is arranged in a front portion 14 of the absorbent core 12. A second adhesive pattern region 13d, formed by two adjacent and uniform adhesive stripes 17, is arranged at a rear portion 16 of the absorbent core 12. A third adhesive pattern region 13b is formed by three adjacent and uniform adhesive stripes 17 arranged at the frontmost part of the central portion 15 of the absorbent core 12. The adhesive stripes 17 of the third adhesive pattern region 13b is in this example uniform to the adhesive stripes 17 arranged in the first adhesive pattern region 13a. The central portion 15 of the absorbent core 12 further comprises a fourth adhesive pattern region 13c, formed by two adjacent and uniform adhesive stripes 17. The adhesive stripes 17 of the fourth adhesive pattern region 13c is in this example uniform to the adhesive stripes 17 arranged in the second adhesive pattern region 13d.

The length of the adhesive stripes 17 in transversal direction W across the width of the absorbent product corresponds to the width of the absorbent core in transversal direction W. Each adhesive stripe 17 within each respective adhesive pattern region 13a-d have the same transversal length. The average transversal length of the adhesive stripes 17 is the same in all four adhesive pattern regions 13a-d, whereas the average longitudinal width of the adhesive stripes 17 and the number of adhesive stripes 17 is the same in the first pattern region 13a and third adhesive pattern region 13b and the second pattern region 13d and fourth adhesive pattern region 13c respectively, such that the two first mentioned pattern regions 13a, 13b differ from the two second mentioned pattern regions 13d, 13c.

The longitudinal distance between the adhesive stripes 17 forming each respective adhesive pattern region 13a-d is the same.

The absorbent article in Figure 6 comprises a first fold region 26 arranged between the front portion 14 and the central portion 15 of the absorbent core 12 and a second fold region 27 between the central portion 15 and the rear portion 16 of the absorbent core 12. The first fold region 26 coincide with the adhesive free region at the transition between the first adhesive pattern region 13a and the third adhesive pattern region 13b, and the second fold region 27 coincide with the adhesive free region at the transition between the second adhesive pattern region 13d and the fourth adhesive pattern region 13c.

The longitudinal distance of adhesive free fold regions 26, 27 in the example shown in Figure 6 is the same but could also differ from each other according to one aspect of the disclosure.

## Claims

1. An absorbent article (11), such as a sanitary napkin or an incontinence protector, having a length extending in a longitudinal direction along a longitudinal axis (L) and a width extending in transversal direction along a transversal axis (W) and having a body-facing surface and a garment-facing surface, the absorbent article (11) comprising
a top sheet, a back sheet, and an absorbent core (12) arranged between the top sheet and the back sheet, and
an adhesive applied to the garment facing side of the back sheet as adhesive stripes (17) oriented along the transversal axis (W), wherein the adhesive stripes (17) extend over the transversal width of the absorbent core (12),
wherein the absorbent article is intended to be folded along transversal imaginary fold lines (28, 29), wherein a first imaginary fold line (28) is arranged in a first fold region (26) positioned between two adhesive stripes (17), and a second imaginary fold line (29) is arranged in a second fold region (27) positioned between two adhesive stripes (17), and wherein no adhesive is present at the first and second fold regions (27),
wherein respective longitudinal widths of the first fold region (26) and second fold region (27) are larger than the average longitudinal distance between the adhesive stripes (17), and the width of the first fold region (26) differs from the width of the second fold region (27), and
wherein at least one of the first and second fold regions (26, 27) has a width of at least 4mm, preferably 5-15mm.

2. Absorbent article according to claim 1, wherein each adhesive stripe (17) extends beyond the transversal width of the absorbent core (12) by no more than 10% of the transversal length of the adhesive stripe (17).

3. Absorbent article according to any of the preceding claims, wherein 30-70%, preferably 45-65%, of the absorbent core area is covered by the adhesive stripes (17).

4. Absorbent article according to any of the preceding claims, wherein the length of the adhesive stripes (17) is 70-120%, preferably 80 - 110 %, and more preferably 90 - 105 %, of the width of the absorbent core (12) in transversal direction.

5. Absorbent article according to any of the preceding claims, wherein the shape of the absorbent core (12) is non-rectangular and/or wherein the transversal width of the absorbent core (12) varies along the longitudinal length of the absorbent core (12).

6. Absorbent article according to any of the preceding claims, wherein the adhesive stripes (17) form at least three adhesive pattern regions (13a-d) and a first adhesive pattern region (13a) is formed by adhesive stripes (17) arranged at a front portion (14) of the absorbent core (12), a second adhesive pattern region (13d) is formed by adhesive stripes (17) arranged at a rear portion (16) of the absorbent core (12), and a third adhesive pattern region (13b) is formed by adhesive stripes (17) arranged at a central portion (15) of the absorbent core (12), and the average transversal length of the adhesive stripes (17) and/or the average longitudinal width of the adhesive stripes (17) and/or the number of adhesive stripes (17) differ between at least two of the adhesive pattern regions (13a-d), wherein each adhesive stripe (17) has an adhesive stripe area, and the sum of the adhesive stripe areas within one adhesive pattern region (13a-d) differs by no more than 19%, preferably no more than 10%, from the sum of the adhesive stripe areas within another adhesive pattern region (13a-d).

7. Absorbent article according to claim 6, wherein the adhesive stripes (17) arranged within the same adhesive pattern region (13a-d) are uniform.

8. Absorbent article according to claim 6 or claim 7, wherein each adhesive stripe (17) has a length extending along the transversal axis (W), a width extending along the longitudinal axis (L), and wherein the lengths of the adhesive stripes (17) arranged within the same adhesive pattern region (13a-d) differ by no more than 10% and the widths of the adhesive stripes (17) arranged within the same adhesive pattern region (13a-d) differ by no more than 10%, and/or wherein the total surface weight of adhesive applied in each respective pattern adhesive region (13a-d) is the same.

9. Absorbent article according to any of claims 6 to 8, wherein the adhesive stripes (17) are arranged to overlap the absorbent core (12) such that at least 90% of each adhesive stripe area is overlapping the absorbent core (12).

10. Absorbent article according to any of claims 6 to 9, a fourth adhesive pattern region (13c) is arranged in a central portion (15) of the absorbent core (12), and wherein the fourth adhesive pattern region (13c) is arranged closer to the rear portion (16) than the third adhesive pattern region (13b), preferably wherein the adhesive stripes (17) of the first adhesive pattern region (13a) has the same length and the same width as the adhesive stripes (17) in the third adhesive pattern region (13b), and wherein the adhesive stripes (17) of the second adhesive pattern region (13d) has the same length and the same width as the adhesive stripes (17) in the fourth adhesive pattern region (13c) and/or preferably wherein the number of the adhesive stripes (17) in the first adhesive pattern region (13a) is the same as the number of the adhesive stripes (17) in the third adhesive pattern region (13b), and the number of the adhesive stripes (17) in the second adhesive pattern region (13d) is the same as the number of the adhesive stripes (17) in the fourth adhesive pattern region (13c).

11. Absorbent article according to any of claims 6 to 10, wherein each adhesive pattern region (13a-d) comprises at least two adhesive stripes (17), and/or wherein each adhesive pattern region (13a-d) comprises no more than 3 adhesive stripes.

12. Absorbent article according to any of claims 6 to 11, wherein the longitudinal distance between the adhesive stripes (17) forming an adhesive pattern region (13a-d) is the same, and/or wherein the longitudinal distance between the adhesive stripes (17) is longer in the second adhesive pattern region (13d) than in the first adhesive pattern region (13a).

13. Absorbent article according to any of claims 6 to 12, wherein the absorbent core (12) is non-continuous in transversal direction in the rear portion (16) of the absorbent article such as to form an intermittent core-free part, and wherein the adhesive stripes (17) of the second adhesive pattern region (13d) covers both the absorbent core (12) and the intermittent core-free part of the rear portion (16) in transversal direction.

14. Absorbent article according to any of claims 6 to 13, wherein the average width of the absorbent core (12) differs between at least two of the front portion (14), the rear portion (16), and the central portion (15).

15. Absorbent article according to any of claims 6 to 14, wherein the absorbent article is provided with a cover material covering at least the adhesive pattern regions (13a-d), the cover material comprising material having a basis weight of 10-25 gsm.
